# EUROPEAN PATENT APPLICATION

(11) **EP 4 042 952 A1**
(43) Date of publication of application: **17.08.2022**
(21) Application number: 22154607.0
(22) Date of filing: 01.02.2022
(51) Int. Cl.: A61B 17/128, A61B 17/29

(54) **LAPAROSCOPIC CLIPPING MACHINE FOR APPLICATION OF SURGICAL CLIPS ON TISSUE STRUCTURES**

(30) Priority: 10.02.2021 PL 43691821
(71) Applicant: "Konmex" Spólka Z Ograniczona Odpowiedzialnoscia, 00-716 Warsaw (PL)
(72) Inventor: Wawryniuk, Grzegorz, 02-495 Warsaw (PL)
(74) Representative: Kondrat, Mariusz

(57) **Abstract**

Laparoscopic clipping machine for application of surgical clips to tissue structures, comprising handgrip, stem integrated in the handgrip, jaws mounted at the end of the stem, trigger lever pivotally mounted with respect to the handgrip, tendon assembly providing a movable coupling between the trigger lever and the jaws, wherein said tendon assembly extends along the longitudinal axis of the stem, wherein for the initial position of the trigger lever the jaws are in an initially open position, **characterised in that** it is provided with initial position change mechanism (7) of the trigger lever (2), which comprises a first stop element (26A) and a second stop element (26B) which are adjustable, wherein the trigger lever (2) is provided with a third stop element (2B), which is in contact with the first stop element (26A) or the second stop element (26B), when the jaws (5) are in the initial position.

## Description

The subject of the invention is the laparoscopic clipping machine adapted for application of different sizes of clamps - surgical clips, on tissue structures.

In surgical practice, endoscopic surgical clipping machines are used, where the end effector for clip application is a moving jaw that opens and closes. State of the art clipping machines for clips application on tissue structures comprises two main elements:
the handgrip equipped with a movable trigger lever and the endoscopic part - the stem, usually in the form of a tube terminated by an effector in the form of a pair of movable jaws. The movable jaws are mounted at the end of the stem, and the movement triggering mechanism of the jaws comprises at least one pusher or tendon, usually axially positioned within the stem, which is moved by a trigger mechanism. The trigger mechanism usually comprises a trigger lever, by the movement of which the pusher moves and the jaws close. Spring located in the handgrip causes the triggering lever to return to its initial position and the jaws to open. The mechanism that causes the movement of the jaws between the two positions - initial open and closed - does not allow the size of the clips to be changed to larger or smaller as the jaws move within a fixed range of opening, which means that a particular clipping machine is only suitable for one type of clip. Thus, in the case of a surgical procedure where clips of different sizes are needed, several clipping machines are required instead of one.

From the description of the invention EP3560442A1, there is a laparoscopic clipping machine known having a stem, a handgrip, a trigger lever and a mechanism which transmits the movement of the trigger lever to the jaws through a pusher located in the stem of the clipping machine, wherein the jaws are coupled to the trigger lever so that when the jaws are clamped, the pusher is pushed. The jaws of the disclosed clipping machine are provided with catches between which a clip is inserted, the clip being clamped by tightening the trigger lever against the handle, wherein the clipping machine is provided with a lever mechanism for moving the tendon.

Due to the frequent need to apply different sized clips during a single surgical procedure, clipping machines that provide the ability to apply different sized clips are in demand. Since many different dedicated laparoscopic instruments may be needed during surgery, there is an expectation that new laparoscopic instruments should be simple, intuitive to use and ergonomic. Due to the multiple actions required during surgery, including the need for different clip sizes, there is a demand that the initial jaw opening can be changed without changing the instrument, and that the performance of the clipping machine is repetitive regardless of the size of the clip used.

The object of the invention is a laparoscopic clipping machine with a simple design, which provides the possibility to quickly change the initial spacing of the jaws and therefore to change the initial spacing of the catches, in order to adapt the clipping machine to accommodate different size clips, thereby reducing the duration of the surgical procedure.

The subject matter of the invention is the laparoscopic clipping machine for applying of surgical clips to tissue structures comprising a handgrip, a stem integrated into the handgrip, jaws mounted at the end of the stem, a trigger lever pivotally mounted relative to the handgrip, a tendon assembly providing a movable coupling between the trigger lever and the jaws, wherein said tendon assembly extends along the longitudinal axis of the stem, wherein for the initial position of the trigger lever the jaws are in an initially open position. The clipping machine is characterized in that it is provided with a mechanism for changing the initial position of the trigger lever, which comprises a first stop element and a second stop element which are adjustable, wherein the trigger lever is provided with a third stop element which is in contact with the first stop element or the second stop element when the jaws are in the initial position.

The clipping machine is characterised in that the mechanism for changing the initial position of the trigger lever comprises an initial position change knob of the trigger lever, which is provided with a first stop surface comprising a first stop element and a second stop surface comprising a second stop element.

The first stop face and the second stop face may be located perpendicular to the longitudinal axis of the stem.

The clipping machine is characterised in that the initial position change knob of the trigger lever is in the form of a ring, the inner cylindrical surface of which is positioned on a body integrated with the handgrip.

The trigger lever initial position change mechanism may be provided with a latch to lock the position of the initial position change knob of the trigger lever.

The latch may be in the form of a ball catch, wherein the initial position change knob of the trigger lever may have recesses for the latch made on the inner cylindrical surface.

The initial position change knob of the trigger lever can be provided with an indicator to indicating the selected initial jaw spacing.

The clipping machine is characterised in that the third stop element is a cylindrical stop surface.

The third stop element may be a stop pin or a screw element located on the trigger lever.

The clipping machine is characterised in that the rotary joint of the trigger lever and the third stop element are located on opposite sides of the longitudinal axis of the stem.

The clipping machine is characterized in that the trigger lever is provided with a pulling joint pin located between the trigger lever rotary joint and the third stop element, the pulling joint being coupled to the tendon assembly.

An advantage of the laparoscopic clipping machine according to the invention is that the provided knob for changing the initial spacing of the jaws, in addition to its primary function, acts as an indicator for the jaw spacing selection and, furthermore, enables audible and tactile recognition of the execution of the spacing selection action. Thus, the operator can control the change in jaw spacing visually, aurally and tactually.

A simple knob is used to change the spacing of the jaws, which is a very ergonomic solution. The operator has one knob to change the angle position of the jaws and right next to it a knob to change the initial position of the trigger lever, and by coupling with the jaws, said knob is used to change the initial spacing of the jaws. The simplicity of the mechanism ensures intuitive adjustment of the knob, and recognition of the expected knob position is obvious. The simplicity guarantees constant precision and durability of the tool.

The object of the invention is described in more detail with reference to the embodiment shown in the figure, in which:

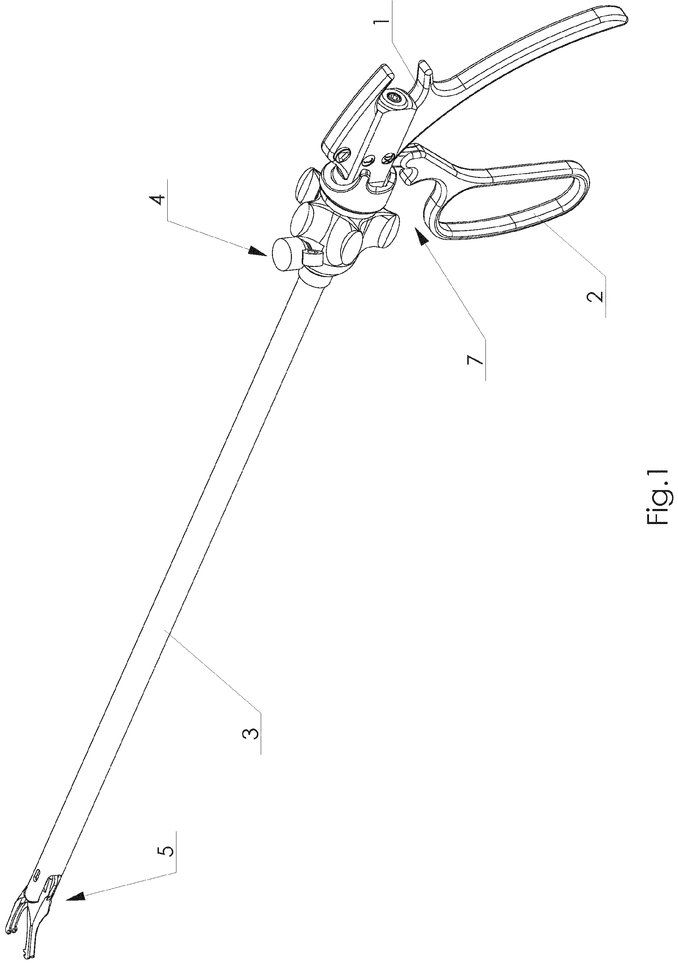

Fig. 1 shows a perspective view of the laparoscopic clipping machine,
Figs. 2a, 3a shows the clipping machine having mechanisms in the initial position for the smaller clip,
Figs. 2b, 3b shows the clipping machine having the mechanisms in the initial position for the larger clip, and
Fig. 4 shows the clipping machine in the position after the trigger lever has been tightened.

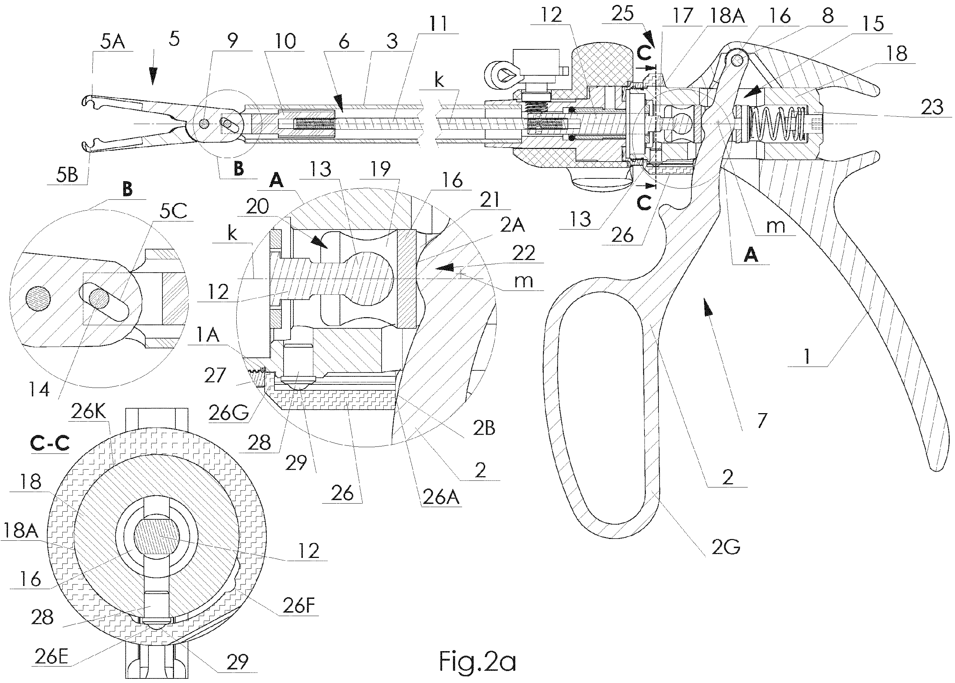

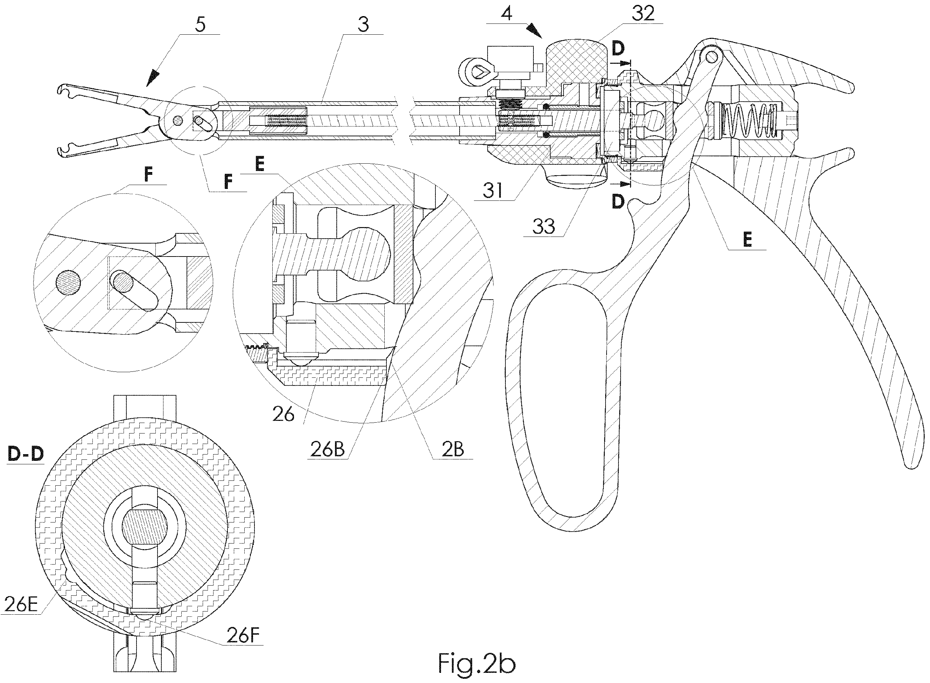

The laparoscopic clipping machine comprises a handgrip 1, a trigger lever 2, a stem 3, a angular position change mechanism 4 of the jaws, jaws 5 and a tendon assembly 6. The clipping machine is provided with a mechanism for changing the initial position 7 of the trigger lever 2. The trigger lever 2 is rotatably mounted on a rotary joint 8 of the trigger lever 2 arranged in the handgrip 1 (Fig. 2a). Jaws 5 are pivotally mounted on a joint 9 at the distal end of stem 3, wherein the jaws 5 are moved by means of a tendon assembly 6 (Figs. 2a, 2b). The tendon assembly 6 comprises a distal tip 10 at the side of the jaws 5, tendon 11 and a proximal tip 12 at the side of the handgrip 1. Tendon assembly 6 is designed to move along the longitudinal axis k of the stem 3, which may be the geometrical axis of both the stem 3 and the tendon 11.

The distal tip 10 is connected to the tendon 11 by means of a thread. The proximal tip 12 is also connected to the tendon 11 by means of a thread. The tendon assembly 6 movably couples the jaws 5 to the trigger lever 2, wherein the trigger mechanism 15 comprises the trigger lever 2, the slide 16, the spring 23 and the tendon assembly 6. The proximal end 12 of the tendon assembly 6 is provided with a head 13, which is moved by the trigger lever 2 by means of the slide 16, rotation of the trigger lever 2 towards the handgrip 1 causes the jaws 5 to clamp. The jaws 5 are provided with catches 5A and 5B in which the clip tips are inserted. The jaws 5 are provided with openings 5C and 5D in which a bolt 14 attached to the further end 10 moves, the movement of the bolt 14 causing the jaws 5 to rotate.

The slider 16 of the trigger mechanism 15 moves in the opening 17 of the body 18 solidly connected to the handgrip 1 and forming an integral part of the handgrip 1. The slider 16 is provided with a first opening 19 having an axis perpendicular to the longitudinal axis k of the stem 3. Inside the opening 19 a head 13 is positioned, wherein the opening 19 is open on the side of the tendon assembly 6. The head 13 along with the opening 19 forms a joint 20 of the proximal end 12 of the tendon 11. In addition, inside the slider 16, a second opening 21 is provided, in which the pin 2A of the trigger lever 2 is located, wherein the pin 2A may have a cylindrical shape with an axis m perpendicular to the longitudinal axis k of the stem, the second opening 21 may have a rectangular cross-section. The second opening 21 together with the pin 2A forms the pulling joint 22. Any other shape of the pulling joint 22 is possible. The movement of the pivotally mounted trigger lever 2 through the pulling joint 22 forces the linear movement of the slider 16 and the tendon assembly 6 according to the longitudinal axis k. The trigger mechanism 15 is provided with a spring 23, which is a compression spring and is mounted in the body 18 so that it forces the movement of the slider 16 in the direction to the jaws 5. The hand-derived pressure on the trigger lever 2 causes the movement of the slider 16 and the compression of the spring 23, the release of the pressure causes the trigger lever 2 to return to its initial position under the action of the spring 23.

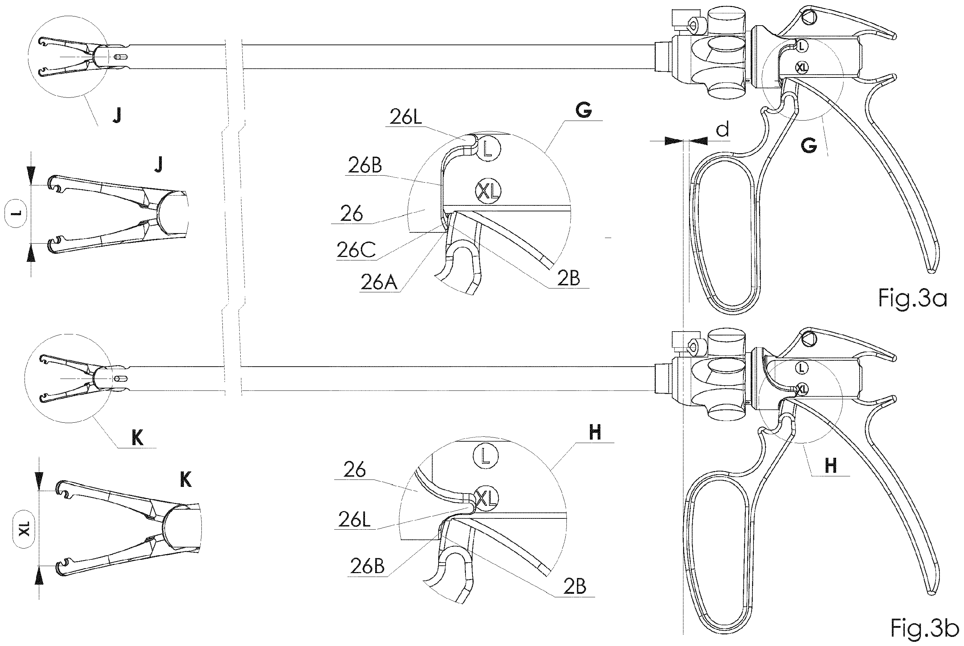

Initial position change mechanism 7 of the trigger lever 2, together with the tendon assembly 6, comprises an initial spacing change mechanism 25 of the jaws 5. The initial spacing of the jaws 5, i.e. the distance between the catches 5A and 5B, is directly related to the change of the initial position of the trigger lever 2. The initial position change mechanism 7 of the trigger lever 2 is provided with an initial position change knob 26 of the trigger lever 2, i.e. it is simultaneously an initial spacing change knob of the jaws 5. Initial position change knob 26 of the trigger lever 2 is in the form of a ring having an inner cylindrical surface 26K rotatably mounted on the cylindrical surface 18A of the body 18 of the handgrip 1. The initial position change knob 26 of the trigger lever 2 may be provided with a first stop element and a second stop element, which are adjustable so as to support the trigger lever, i.e. to determine the initial position of the trigger lever 2. The stop elements of the initial position change knob 26 of the trigger lever 2 may be of any form, for example they may be stop bolts. The third stop element located on the trigger lever 2 may be of any form. In the embodiment of the initial position change knob 26 of the trigger lever 2 shown, the first stop element 26A has the form of a stop surface 26A and the second stop element 26B has the form of a second stop surface 26B (also shown in Figs. 3a and 3b), while the third stop surface 2B has the form of a convex surface, wherein the convexity is directed towards the jaws 5. The third stop surface 2B may be in the form of a cylindrical surface, the third stop surface 2B may be in the form of any protrusion on the trigger lever 2 on the stem 3 side. For example, a stop bolt pressed into the trigger lever 2 or an element screwed into the trigger lever 2 may be used. In the initial position of the trigger lever 2, the third stop surface 2B on the trigger lever 2 may be pressed against the first stop surface 26A or the second stop surface 26B on the initial position change knob 26 of the trigger lever 2 under the action of the spring 23. In the embodiment shown, the support of the trigger lever 2 against the first stop surface 26A is adapted to open the jaws 5 to set to an opening suitable for the size L clips (Figs. 2a and 3a). The support of the trigger lever 2 against the second stop surface 26B ensures the maximum pivoting of the trigger lever 2 and is adapted to open the jaws 5 to set to an opening suitable for size XL clips (Figs. 2b and 3b). The distance indicated as d in Fig. 3a between the first stop surface 26A and the second stop surface 26B in a direction parallel to the longitudinal axis k enables to obtain two different initial positions of the trigger lever 2, wherein the repositioning of the stop surface 26A and 26B is carried out by rotating the knob 26. Furthermore, the initial position change knob 26 of the trigger lever 2 has an intermediate surface 26C connecting the first stop surface 26A and the second stop surface 26B, which provides a smooth transition between these surfaces. The knob 26 is provided with an indicator 26L, which indicates the selected one of the possible spacings of the jaws 5 i.e. spacing L or XL, the indicator 26L can be positioned in front of one of the descriptions L or XL on the handgrip 1 when the knob 26 is locked as shown in Figures 3a and 3b. The indicator 26L is in the form of a rounded finger adjacent the cylindrical surface 18A of the body 18. The axial position of the initial position change knob 26 of the trigger lever 2 is fixed by means of a flange 26G, which is resting on the groove 1A of the handgrip 1 and is located along the longitudinal axis k of the stem 3 by a nut 27. The initial position change mechanism 7 of the trigger lever 2 is provided with a latch 28 having a latching element 29 in the form of a ball, which is arranged to lock the knob 26. For the spacing L, the latching element 29 slides into the recess 26E, and for the spacing XL, the latching element 29 slides into the recess 26F.

The trigger mechanism 15 is designed so that the tightening of the lever 2 against the handgrip 1 creates movement of the tendon assembly 6 in a direction towards the handgrip 1. The rotary joint 8 of the trigger lever 2 and the third stop surface 2B of the trigger lever 2 are located on opposite sides with respect to the longitudinal axis k of the stem 3 and on opposite sides of the pulling joint 22. Furthermore, the gripping part 2G of the trigger lever 2 and the rotary joint 8 of the trigger lever 2 are similarly located on opposite sides with respect to the longitudinal axis k of the stem 3 and the pulling joint 22.

The clipping machine is provided with angular position change mechanism 4 of the jaws 5, which comprises a sleeve 31 and an angular position change knob 32 of the jaws 5. The stem 3 is fixedly connected to the sleeve 31, which remains in contact and rotates with the inner track of the bearing 33. Rotation of the angular position change knob 32 of the jaws 5 causes rotation of the stem 3 and the jaws 5 fixed thereon.

As shown in Figs. 2a and 2b as well as in Figs. 3a and 3b, the trigger lever 2 and the jaws 5 are in their initial position, wherein in Fig. 2a the third stop surface 2B on the trigger lever 2 rests against the first stop surface 26A on the knob 26 and the spacing of the jaws 5 is adapted for size L clips, the indicator 26L of the knob 26 indicates the selected size L (Fig. 3a), respectively, whereas in Fig. 2b the knob 26 is rotated so that the indicator 26L shows the selected size XL (Fig. 3b), the third stop surface 2B of the trigger lever 2 rests against the second stop surface 26B on the knob 26, and the spacing of the jaws 5 is adapted for size XL clips.

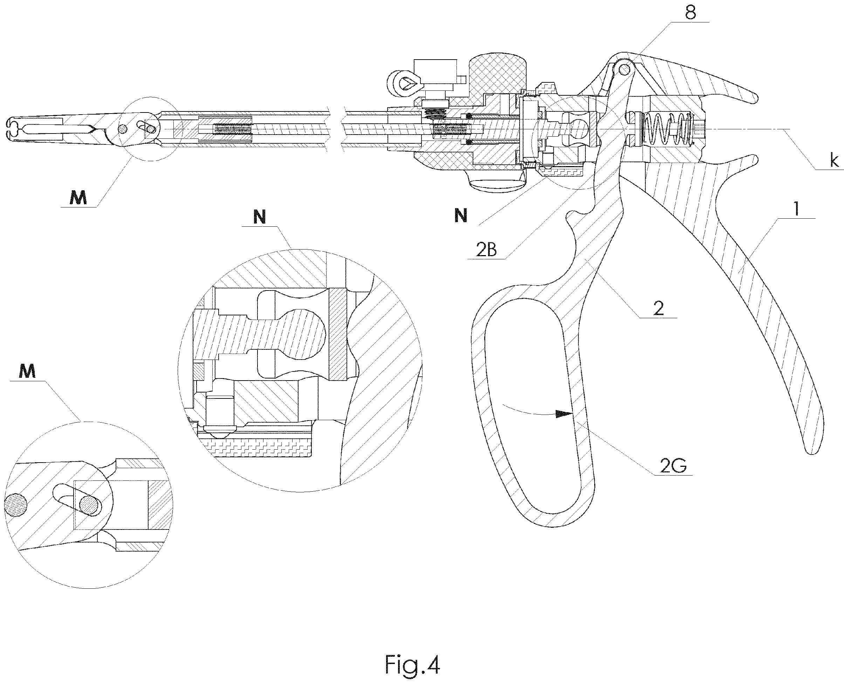

Fig. 4 shows the clipping machine in the position as when the clip is clamped, the trigger lever 2 is tightened to the maximum level against the handgrip 1.

The change in the spacing of the jaws 5 is carried out by gripping the handle 1 and the trigger lever 2, wherein the trigger lever 2 and the handle 1 must be tightened slightly so as to rotate the trigger lever 2 towards the handgrip 1, which will cause the jaws 5 to close. Then, with the other hand or thumb, it is necessary to turn the initial position change knob 26 of the trigger lever 2 changing the initial spacing of the jaws 5 into a position according to the size of the L or XL clips used, the 26L indicator being set in front of one of the L or XL descriptions on the body 18. The setting of the knob 26 in one of the possible positions is accompanied by an audible locking of the latch 28 in one of the recesses 26E or 26F, wherein the locking of the latch 28 can be felt with the hand, since the latching causes minimal vibration of the handle and the knob. The operator of the clipping machine according to the invention, therefore, has a threefold confirmation of having made a change in the setting of the initial opening of the jaws of the clipping machine.

The angular position change mechanism 4 of the jaws 5 is independent of the initial spacing change mechanism 25 of the jaws 5. The angular position change mechanism 4 of the jaws 5 may be provided with a latching element.

## Claims

1. Laparoscopic clipping machine for application of surgical clips to tissue structures, comprising
- handgrip,
- stem integrated in the handgrip,
- jaws mounted at the end of the stem,
- trigger lever pivotally mounted with respect to the handgrip,
- tendon assembly providing a movable coupling between the trigger lever and the jaws,
wherein said tendon assembly extends along the longitudinal axis of the stem,
wherein for the initial position of the trigger lever the jaws are in an initially open position,
**characterised in that**
it is provided with initial position change mechanism (7) of the trigger lever (2), which comprises a first stop element (26A) and a second stop element (26B) which are adjustable, wherein the trigger lever (2) is provided with a third stop element (2B), which is in contact with the first stop element (26A) or the second stop element (26B), when the jaws (5) are in the initial position.

2. The clipping machine according to claim 1, **characterised in that**, initial position change mechanism (7) of the trigger lever (2), comprises initial position change knob (26) of the trigger lever (2), which is provided with a first stop surface comprising a first stop element (26A) and a second stop surface comprising a second stop element (26B).

3. The clipping machine according to claim 2, **characterised in that**, first stop face (26A) and the second stop face (26B) may be located perpendicular to the longitudinal axis (k) of the stem (3).

4. The clipping machine according to claim 2 or 3, **characterised in that**, initial position change knob (26) of the trigger lever (2), is in the form of a ring, the inner cylindrical surface (26K) of which is positioned on a body (18) integrated with the handgrip (1).

5. The clipping machine according to claim 2 or 4, **characterised in that**, initial position change mechanism (7) of the trigger lever (2) is provided with a latch (28) to lock the position of the initial position change knob (26) of the trigger lever (2).

6. The clipping machine according to claim 5, **characterised in that**, the latch (28) is in the form of a ball latch.

7. The clipping machine according to claim 4 or 6, **characterised in that**, initial position change knob (26) of the trigger lever (2) has recesses (26E, 26F) for the latch (28) made on the inner cylindrical surface (26K).

8. The clipping machine according to claim 4 or 7, **characterised in that**, initial position change knob (26) of the trigger lever (2) is provided with an indicator (26L) for indicating which initial jaw spacing (5) is selected.

9. The clipping machine according to claim 1 or 8, **characterised in that**, the third stop element (2B) is a cylindrical stop surface.

10. The clipping machine according to claim 1 or 8, **characterised in that**, the third stop element (2B) is a stop bolt or a screw element located on the trigger lever (2).

11. The clipping machine according to any one of the preceding claims, **characterised in that**, the rotary joint (8) of the trigger lever (2) and the third stop element (2B) are located on opposite sides of the longitudinal axis (k) of the stem (3).

12. The clipping machine according to any one of the preceding claims, **characterised in that**, the trigger lever (2) is provided with a pulling joint (22) pin (2A) located between the trigger lever (2) rotary joint (8) and the third stop element (2B), wherein the pulling joint (22) being coupled to the tendon assembly (6).
